# EUROPEAN PATENT SPECIFICATION

(11) **EP 2 568 970 B1**
(45) Date of publication and mention of the grant of the patent: **05.12.2018**
(21) Application number: 11723895.6
(22) Date of filing: 10.05.2011
(51) Int. Cl.: A61K 9/32, A61K 9/28, A61K 31/4545

(54) **STABLE PHARMACEUTICAL FORMULATIONS CONTAINING AN ANTIHISTAMINIC**
STABILE PHARMAZEUTISCHE FORMULIERUNGEN MIT EINEM ANTIHISTAMINIKUM
FORMULATIONS PHARMACEUTIQUES STABLES CONTENANT UN ANTIHISTAMINIQUE

(30) Priority: 10.05.2010 EP 10162403
(43) Date of publication of application: 20.03.2013
(73) Proprietor: Laboratorios Lesvi, S.L., 08970 Sant Joan Despí - Barcelona (ES)
(72) Inventor: DÍEZ MARTÍN, Ignacio, E-08970 Sant Joan Despi (Barcelona) (ES); PABLO ALBA, Pablo, E-08970 Sant Joan Despi (Barcelona) (ES); ÚBEDA PÉREZ, Carmen, E-08970 Sant Joan Despi (Barcelona) (ES)
(74) Representative: ABG Intellectual Property, S.L.
(86) International application number: PCT/EP2011/057560
(87) International publication number: WO 2011/141483

(56) References cited:
- EP-A1- 2 269 586
- EP-A2- 1 728 513
- WO-A1-2008/138563
- CA-A1- 2 571 933
- US-A1- 2008 118 555

## Description

### FIELD OF THE INVENTION

This invention relates to stable solid oral pharmaceutical compositions of 8-chloro-6,11-dihydro-11-(4-piperidylidene)5H-benzo[5,6]cyclohepta[1,2-b]pyridine, also known as descarboethoxyloratadine (desloratadine or DCL) consisting of a tablet core and a filmogenic coating.

### BACKGROUND OF THE INVENTION

Desloratadine is a tri-cyclic antihistamine, used to treat allergies and sold under the brand names of Clarinex and Aerius 5 mg coated tablets. Desloratadine is known to be a compound prone to undergo oxidation and decomposition by acidic excipients, which generate impurities such as N-formyldesloratadine, deschlorodesloratadine and dihydrodesloratadine.

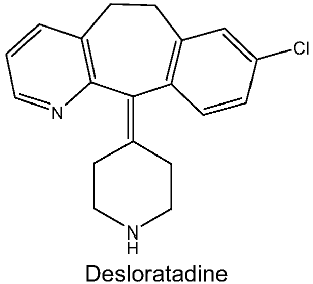

WO0002560 describes Desloratadine and its pharmaceutical compositions comprising Desloratadine and a Desloratadine protective amount of a pharmaceutical acceptable salt such as calcium dibasic phosphate and at least one disintegrant. WO0002560 also addresses the issues of Desloratadine when formulated with acidic excipients. The patent also comments on the instability and rapid discoloration of formulations comprising Desloratadine, mannitol and Mg stearate. In order to prevent or reduce the generation of impurities, pharmaceutical compositions comprising Desloratadine, a Desloratadine protective amount of a calcium, magnesium or aluminium salt, (particularly, dibasic calcium phosphate) and at least one disintegrant were provided.

WO2008138563 describes desloratadine tablets containing lactose and Opadry II coating.

WO9834614 patent application reports the reactivity between lactose and Desloratadine. In order to avoid said interaction, patent application WO9834614 discloses stable desloratadine-containig formulations free of reactive components such as lactose and mono or disaccharides.

Furthermore, patent application WO9834614 also provides a lactose free pharmaceutical composition for the treatment of histamine disorders, prepared by granulation methods. An important second aspect of the invention is to provide a pharmaceutical composition for the treatment of histamine-induced disorders comprising large particles of Desloratadine, with the purpose of decreasing the particles surface area and therefore, reducing interactions between DCL and reactive excipients, such as lactose. The Desloratadine compositions described in this patent comprised particles size of 250 µm or greater.

Application patent WO2007140987 provides a stable formulation containing Desloratadine without the addition of a basic salt, while still using acidic components, although the use of lactose in said formulations is avoided.

WO2006008512 also provides pharmaceutical compositions comprising DCL, which does not discolour, without the need to resort to the use of a basic salt.

PCT application WO2006008512 deals with the incompatibility of DCL with excipients such as lactose and Mg stearate, which degrade DCL, turning the pharmaceutical composition into a pink colour. As a result of that, the use of lactose and Mg stearate, in the formulations herein disclosed, was avoided.

As explained above, Desloratadine has been reported to be unstable in compositions comprising excipients such as lactose or mono or disaccharides limiting significantly the number excipients, which could be used to develop stable formulations. A stable pharmaceutical composition of Desloratadine using standard and industrially viable excipients is still in need to be developed.

### SUMMARY OF THE INVENTION

The invention relates to stable oral solid pharmaceutical compositions of desloratadine as defined in the claims.

The pharmaceutical compositions herein disclosed have a good photostability, and they comply with the pharmacopeia requirements.

The authors of the present invention have found surprising effects on the stability of the desloratadine in the presence of lactose when the formulation is coated with a polymer having filmogenic properties.

There are disclosed (but are not a part of the present invention) stable lactose-containing oral solid pharmaceutical compositions of DCL, comprising small particle size of Desloratadine. The small particle size of DCL have an "average particle size", D(50), below 20 µm. Unexpectedly, the authors of this invention have found that stable lactose-containing oral solid pharmaceutical compositions of DCL, can be achieved by using particles of desloratadine having a D(50) below 20 µm.

The lactose-containing pharmaceutical composition of Desloratadine of the present invention, initially contains less than 0.1 % by weight (measured by HPLC) of total degradation impurities (including N-formyldescarbonyl ethoxyloratadine). The percentage of total degradation impurities is also maintained below 0.1% by weight of the total weight of the tablet after 1 month at 40 °C/75 % HR.

A further aspect of the present invention is to provide a process by direct compression for preparing the tablets as defined in the claims.

Moreover, the present invention relates to the stable pharmaceutical compositions of Desloratadine herein described, for use in the treatment or prophylaxis of allergic rhinitis and other histamine disorders.

### DETAILED DESCRIPTION OF THE INVENTION

The term "average particle size", D(50), as used herein refers to the volume mean diameter, of particles. D(x) value indicates that a certain percentage X of total volume of the particles has a size below certain limit. For instance, a D(50) of 20 µm represents that 50% of the total volume of a Desloratadine sample correspond to particles having a diameter below 20 µm. The diameter and volume mean diameter can be measured by light scattering using a Malvern-Mastersizer Apparatus MS 2000. Particle size is determined by measuring the angular distribution of laser light scattered by a homogeneous suspension of particles. The size distribution is determined from the light scattering data using the theory of light scattering developed by Gustav Mie.

The term a core tablet blend or a core tablet, in accordance with this invention, comprises a mixture of active ingredient and acceptable excipients compressed to form a compressed core tablet, which can be further film coated.

By stable composition is meant a composition which is in compliance with physicochemical parameters. For instance, compositions which do not show any significant discoloration and which are substantially free of DCL decomposition products (the content of drug does not fall below 95% by weight of the active ingredient, and dissolution is not less than 80 % by weight of the active ingredient during the specified shelf life). DCL decomposition products may include impurities such as N-formyl desloratadine, deschlorodesloratadine and dihydrodesloratadine.

A significant number of patents and publications have been disclosed addressing this issue and providing alternative formulations, which avoid the use of lactose and mono or disaccharides.

Surprisingly, the inventors have found that stable pharmaceutical compositions of Desloratadine, comprising excipients which have been reported as being incompatible with DCL such as lactose, mono or disaccharides and Mg stearate, can be prepared having very low levels of DCL degradation products when the formulation is coated with a polymer having filmogenic properties.

Lactose-containing oral solid pharmaceutical compositions of Desloratadine may as well comprise an additional diluent selected from the group of microcrystalline cellulose; mannitol and isomaltose. Lactose was used as diluent, preferably in an amount from 12% to 50%.

Other standard pharmaceutical excipients that may be optionally used include one or more but not limited to diluents, disintegrants and pH modifiers.

In the present invention, "diluent" is taken to mean: saccharides (monosaccharides or oligosaccharides, polysaccharides), and/or their oxidised and/or reduced forms; lactose in its various forms, anhydrous, monohydrate, agglomerated forms or atomised forms; mannitol; cellulose powder, microcrystalline cellulose, silicified microcrystalline cellulose or derivatives of cellulose modified chemically, such as hydroxypropyl cellulose, hydroxypropyl methyl cellulose; maize starch and sucrose. Preferably, additional diluents are selected from the group of microcrystalline cellulose, maize starch, mannitol and isomaltose. Additional acceptable diluents may be present in an amount from 30 % to 65 %.
In the present invention "lubricant" is taken to mean: magnesium stearate, hydrogenated cotton seed oil, sodium stearyl fumarate, magnesium stearate, calcium stearate; glyceryl palmitostearate, talc, stearic acid, glyceryl behenate, fatty acid esters, sodium lauryl sulphate, or mixtures thereof. Preferably a stearate will be used, and more preferably a magnesium stearate. Optionally, more than one lubricant can be present in the tablet core. Suitable lubricants may be present in concentrations from 0.5 to 5 %.
In the present invention "disintegrant" is taken to mean: low-substitution hydroxypropyl cellulose, hydroxyethyl cellulose, crospovidone, croscarmellose, pregelatinised starch, sodium starch carboxymethyl, croscarmellose sodium, soy polysaccharides, and derivatives of casein or mixtures thereof. Suitable disintegrants may be present in concentration from 5 to 30 % w/w of a tablet.
In the present invention "pharmaceutical acceptable pH modifiers" is taken to mean compounds such as phosphates, carbonates, citrates. The pH modifier adjusts and balances the water intake within the tablet and therefore the pH of the media. Pharmaceutical acceptable pH modifiers may be present in concentrations from 0 to 3 % w/w of a tablet.
The tablet core blend comprises between 12-50% w/w (by weight of the total weight of the tablet core) of lactose and anhydrous lactose. Additionally, the tablet core comprises one or more of the following components:
a) 30 % to 65 % w/w of diluents other than lactose
b) 0.5-5% w/w of lubricants
c) 5-30% w/w of disintegrants
d) 0-3% w/w pH modifiers.
being the ratio of the pH modifier to the disintegrant below 0.8 %. Preferably below 0.5%.
The utilisation of a coating with a functional polymer soluble in and aqueous medium, which acts as a filmogenic agent, allows the tablet to have lower permeability to water vapour, thus involving a lower take-up of moisture from the environment, allowing at the same time a suitable release of the active substance without detriment to its bioavailability.

The functional polymer is based on polyvinyl alcohol as filmogenic agent. Suitable coating can be applied to the tablet core at concentrations of 1 to 10% (w/w). Polyvinyl alcohol is a non-toxic synthetic polymer soluble in water, thus making it an appropriate candidate for utilisation in the formulation of the invention.
The coating of the formulation in accordance with the first aspect of the invention can further include one or more of the following excipients: from 10 to 50% of opacifier and colorant, from 5 to 30% of lubricant, from 0.5 to 5% of plasticizer, from 0.1 to 3% of thickening agent and from 0 to 20% of filler agent.
Preferably, the opacifiers and colorants, contained in the coating, can be selected from: titanium dioxide, calcium carbonate, iron oxides, aluminium lakes or mixtures thereof.
Preferably, the plasticizers, contained in the coating, can be selected from: glycerol, polyethylene glycol, sorbitol, soya lecithin, esters of citric acid or mixtures thereof.
Preferably, the lubricants, contained in the coating, can be selected from: stearates, sodium benzoate, stearic acid, talc or mixtures thereof.
Preferably, the thickening agent, contained in the coating, can be selected from: xanthan gum, bentonite, carrageenates, colloidal silica, ethylcellulose, gelatine, maltitol, sucrose or mixtures thereof.

Surprisingly, it has been observed that the utilisation of an aqueous medium for preparation of the coating does not affect the stability of the solid form, with no increase of degradation related substances nor change in the polymorphic form of the Desloratadine being detected.
Preferably, said aqueous medium is water. The present invention nevertheless provides for the presence of organic solvents such as short-chain alcohols, for example, methanol, chlorinated derivatives such as methylene chloride, acetone or mixtures thereof with water.

The coating method comprises: i) providing a tablet core containing desloratadine, lactose in an amount from 12 to 50% by weight of the total weight of the tablet core and pharmaceutically acceptable excipients in solid form; ii) providing a functional polymer that acts as filmogenic agent, said functional polymer being based on polyvinyl alcohol, wherein between 4% w/w and 8% w/w of the total amount of the pharmaceutical composition is the functional polymer; iii) preparing a dispersion of said functional polymer in an aqueous medium; and applying the dispersion obtained in step iii) onto the solid form of step i).
The use of a functional coating that acts as filmogenic agent also permits a reduction in the protection and packaging requirements of the tablet, such as for airtight packaging impermeable to water vapour (for example, glass vials) or wrappings (for example, blister packs) that protect the solid form from the environment.

The lactose-containing oral solid pharmaceutical composition of Desloratadine of the present invention contains less than 0.1 % by weight of total degradation impurities (measured by HPLC). Preferably, below 0.05%. The level of the degradation impurity, N-formyl desloratadine, is also maintained below 0.1 % of the total weight of the tablet. Preferably, below 0.05%.
Moreover, the present inventors have found that the compositions of the invention, which initially contains less than 0.1 % by weight (measured by HPLC) of total degradation impurities, preferably below 0.05% are stable and keep the level of total degradation impurities (including N-formyl desloratadine) below 0.1 % of the total weight of the tablet after 1 month at 40°C/75% HR. Preferably, below 0.05%. The level of the degradation impurity, N-formyl desloratadine, is also maintained below 0.1 % of the total weight of the tablet after 1 month at 40°C/75% HR. Preferably, below 0.05%

A further aspect of the present invention is to provide a process for preparing the oral solid pharmaceutical compositions of the first invention by direct compression methods, avoiding therefore a wet granulation step, which may affect the stability of the Desloratadine in the presence of lactose. The direct compression method consists in making an intimate mixture of the active substance with the excipients in the absence of solvents, and then proceeding to compression. Furthermore, direct compression is fast, involves lesser step prior to compression and includes no solvents, so degradation of the active ingredients during processing is reduced to a larger extend. Direct compression of the above compositions provides very good content uniformity with respect to the active ingredient. Preferably, the different samples assay carried out on the content uniformity falls between 90 to 100% of the average content.
The preparation of stable oral solid pharmaceutical compositions of Desloratadine, described in the first embodiment, was carried out by direct compression as described below:
a) Sieving all components contained in the core tablet blend.
b) Pre-mixing the active ingredient with part of the excipients, preferably the same amount by weight of excipients than the active ingredient is mixed in this step, with the proviso that lactose is not included in this step;
c) Mixing the blend obtained in step b) with the remaining excipients with the exception of the glidants and lubricants;
d) Mixing the glidants with the blend obtained in step c);
e) Mixing the lubricant with the blend obtained in step d);
f) Compressing the blend obtained in step e) to form a core tablet;
g) Film coating the tablet core by spaying over an aqueous or solvent dispersion of a film coating preparation.
When two lubricants are present in the tablet core, the additional lubricant is added in step b).

There are also disclosed (but are not a part of the invention) stable lactose-containing oral solid pharmaceutical compositions of DCL, which comprise small particle size of Desloratadine, wherein D(50) varies from 5 up 50 µm. Preferably, small particle size of DCL have a D(90) below 50 µm, D(50) below 20 µm, and a D(10) below 10 µm.

Desloratadine used in the compositions of the invention can be in polymorphic form I or form II or mixtures thereof as described in WO9901450.

The pharmaceutical compositions of the present invention are for use in the treatment or prophylaxis of allergic rhinitis and other histamine disorders.

Parts and percentages being by weight, unless otherwise indicated.

### EXAMPLES

### Example 1

| **Ingredients of formulation** | mg/Tablet |
|---|---|
| **tablet core** | |
| Desloratadine | 5.0 |
| Anhydrous disodium hydrogen phosphate | 1.0 |
| Maize starch | 5.0 |
| Microcrystralline cellulose (102 grade) | 29.0 |
| Microcrystralline cellulose (200 grade) | 29.5 |
| Lactose (CD grade) | 15.0 |
| Pregelatinized starch | 12.5 |
| Anhydrous colloidal silicon dioxide | 0.5 |
| Hydrogenated cotton seed oil | 1.0 |
| Magnesium stearate | 1.5 |
| Total | 100 |

| **Coating** | |
|---|---|
| Opadry AMB blue 80W30557^{*} | 6.0 |

| | |
|---|---|
| **Opadry AMB blue 80W30557 is a dry dispersion for film coating, based on polyvinyl alcohol* | |

### Manufacturing process

1- Desloratadine, anhydrous disodium hydrogen phosphate, cotton seed oil and 5-10 % of microcrystalline cellulose were sieved and mixed.
2- Afterwards, maize starch, lactose (DC grade), pregelatinized starch and the remaining microcrystalline cellulose were sieved and mixed with the blend obtained in step 1.
3- Anhydrous colloidal silicon dioxide was also sieved and blended with the blend obtained in step 2.
4-Magnesium stearate was passed through a sieve and blended with the blend obtained in step 3.
5-The blend obtained in step 4 was compressed into a core tablet
6-The tablet of step 5 was coated with a 12% w/w aqueous dispersion of Opadry AMB blue, using an automated coating machine. After completion of the coating process the tablet was dried for 15 to 90 min at an inlet air temperature between 30 and 65 °C.

The tablets packed in aluminium blisters were subjected to stability studies. Results of the stability studies are shown below.

| | Initially | 1 month 25°C/60% HR | 1 month 40°C/75% HR |
|---|---|---|---|
| Assay (%) | 102.2 | 101.4 | 102.3 |

| Impurities | | | |
|---|---|---|---|
| N-Formyldesloratadine % | ND | ND | ND |
| Total degradation products % | 0.05 | 0.03 | 0.07 |

| | | | |
|---|---|---|---|
| ND: Not detectable, concentrations below 0.01 %. | | | |

### Example 2

| **Ingredients of formulation** | **mg/Tablet** |
|---|---|
| **Tablet core** | |
| Desloratadine | 5.0 |
| Anhydrous disodium hydrogen phosphate | 1.0 |
| Maize starch | 5.0 |
| Microcrystralline cellulose (102 grade) | 29.0 |
| Lactose (DC grade) | 44.5 |
| Pregelatinized Starch | 12.5 |
| Anhydrous Colloidal silicon dioxide | 0.5 |
| Magnesium stearate | 2.5 |
| Total | 100 |

| **Coating** | |
|---|---|
| Opadry AMB blue 80W30557^{*} | 6.0 |

| | |
|---|---|
| **Opadry AMB blue 80W30557 is a dry dispersion for film coating, based on polyvinyl alcohol* | |

### Manufacturing process

1- Desloratadine, anhydrous disodium hydrogen phosphate, cotton seed oil and 10-15 % of microcrystalline cellulose were sieved and mixed.
2- Afterwards, maize starch, lactose (DC grade), pregelatinized starch and the remaining microcrystalline cellulose were sieved and mixed with the blend obtained in step 1.
3- Anhydrous colloidal silicon dioxide was also sieved and blended with the blend obtained in step 2.
4-Magnesium stearate was passed through a sieve and blended with the blend obtained in step 3.
5-The blend obtained in step 4 was compressed into a core tablet
6-The tablet of step 5 was coated with a 12% w/w aqueous dispersion of Opadry AMB blue, using an automated coating machine. After completion of the coating process the tablet was dried for 15 to 90 min at an inlet air temperature between 30 and 65 °C.

The tablets packed in aluminium blisters were subjected to stability studies. Results of the stability studies are shown below.

| | Initially | 1 month 25°C / 60% HR | 1 month 40°C / 75% HR |
|---|---|---|---|
| Assay (%) | 101.3 | 101.3 | 101.3 |

| Impurities | | | |
|---|---|---|---|
| N-Formyldesloratadine % | ND | ND | ND |
| Total degradation products % | 0.09 | 0.06 | 0.11 |

### Example 3

| **Ingredients of formulation** | mg/Tablet |
|---|---|
| **Tablet core** | |
| Desloratadine | 5.0 |
| Maize starch | 5.0 |
| Microcrystralline cellulose (102 grade) | 29.0 |
| Microcrystralline cellulose (200 grade) | 30.5 |
| Lactose (DC grade) | 15.0 |
| Pregelatinized starch | 12.5 |
| Anhydrous colloidal silicon dioxide | 0.5 |
| Magnesium stearate | 2.5 |
| Total | 100 |

| **Coating** | |
|---|---|
| Opadry AMB blue 80W30557^{*} | 6.0 |

| | |
|---|---|
| **Opadry AMB blue 80W30557 is a dry dispersion for film coating, based on polyvinyl alcohol* | |

### Manufacturing process

1- Desloratadine and 5-10 % of microcrystalline cellulose were sieved and mixed.
2- Afterwards, maize starch, lactose (DC grade), pregelatinized starch and the remaining microcrystalline cellulose were sieved and mixed with the blend obtained in step 1.
3- Anhydrous colloidal silicon dioxide was also sieved and blended with the blend obtained in step 2.
4-Magnesium stearate was passed through a sieve and blended with the blend obtained in step 3.
5-The blend obtained in step 4 was compressed into a core tablet
6-The tablet of step 5 was coated with a 12% w/w aqueous dispersion of Opadry AMB blue, using an automated coating machine. After completion of the coating process the tablet was dried for 15 to 90 min at an inlet air temperature between 30 and 65 °C.

The tablets packed in aluminium blisters were subjected to stability studies. Results of the stability studies are shown below.

| | Initially | 1 month 25°C / 60% HR | 1 month 40°C / 75% HR |
|---|---|---|---|
| Assay (%) | 100.8 | 101 | 102.3 |

| Impurities | | | |
|---|---|---|---|
| N-Formyldesloratadine % | ND | ND | ND |
| Total degradation products % | 0.04 | 0.03 | 0.06 |

### Example 4

| **Ingredients of formulation** | mg/Tablet |
|---|---|
| **Tablet core** | |
| Desloratadine | 5.0 |
| Anhydrous disodium hydrogen phosphate | 1.0 |
| Maize starch | 5.0 |
| Microcrystralline cellulose (102 grade) | 29.0 |
| Mannitol | 29.5 |
| Lactose (CD grade) | 15.0 |
| Pregelatinized starch | 12.5 |
| Anhydrous colloidal silicon dioxide | 0.5 |
| Magnesium stearate | 2.5 |
| Total | 100 |

| **Coating** | |
|---|---|
| Opadry AMB blue 80W30557^{*} | 6.0 |

| | |
|---|---|
| **Opadry AMB blue 80W30557 is a dry dispersion for film coating, based on polyvinyl alcohol* | |

### Manufacturing process

1- Desloratadine, anhydrous disodium hydrogen phosphate, cotton seed oil and 5-10 % of microcrystalline cellulose were sieved and mixed.
2- Afterwards, maize starch, lactose (DC grade), pregelatinized starch and mannitol were sieved and mixed with the blend obtained in step 1.
3- Anhydrous colloidal silicon dioxide was also sieved and blended with the blend obtained in step 2.
4-Magnesium stearate was passed through a sieve and blended with the blend obtained in step 3.
5-The blend obtained in step 4 was compressed into a core tablet
6-The tablet of step 5 was coated with a 12% w/w aqueous dispersion of Opadry AMB blue, using an automated coating machine. After completion of the coating process the tablet was dried for 15 to 90 min at an inlet air temperature between 30 and 65 °C.

The tablets packed in aluminium blisters were subjected to stability studies. Results of the stability studies are shown below.

| | Initially | 1 month 25°C / 60% HR | 1 month 40°C / 75% HR |
|---|---|---|---|
| Assay(%) | 102.6 | 100.2 | 99.9 |

| Impurities | | | |
|---|---|---|---|
| N-Formyldesloratadine % | ND | ND | 0,04 |
| Total degradation products % | 0.03 | 0.02 | 0.06 |

### Example 5

### Composition containing

| **Ingredients of formulation** | **mg/Tablet** |
|---|---|
| **Tablet core** | |
| Desloratadine | 5.0 |
| Anhydrous disodium hydrogen | 1.0 |
| phosphate | |
| Maize starch | 5.0 |
| Microcrystralline cellulose (102 grade) | 29.0 |
| Isomaltosa | 29.5 |
| Lactose (CD grade) | 15.0 |
| Pregelatinized starch | 12.5 |
| Anhydrous colloidal silicon dioxide | 0.5 |
| Magnesium stearate | 2.5 |
| Total | 100 |

| **Coating** | |
|---|---|
| Opadry AMB blue 80W30557 | 6.0 |

### Manufacturing process

1- Desloratadine, anhydrous disodium hydrogen phosphate, cotton seed oil and 5-10 % of microcrystalline cellulose were sieved and mixed.
2- Afterwards, maize starch, lactose (DC grade), pregelatinized starch and isomaltose were sieved and mixed with the blend obtained in step 1.
3- Anhydrous colloidal silicon dioxide was also sieved and blended with the blend obtained in step 2.
4-Magnesium stearate was passed through a sieve and blended with the blend obtained in step 3.
5-The blend obtained in step 4 was compressed into a core tablet
6-The tablet of step 5 was coated with a 12% w/w aqueous dispersion of Opadry AMB blue, using an automated coating machine. After completion of the coating process the tablet was dried for 15 to 90 min at an inlet air temperature between 30 and 65 °C.

The tablets packed in aluminium blisters were subjected to stability studies. Results of the stability studies are shown below.

| | Initially | 1 month 25°C / 60% HR | 1 month 40°C/ 75% HR |
|---|---|---|---|
| Assay(%) | 102.5 | 101 | 101.7 |

| Impurities | | | |
|---|---|---|---|
| N-Formyldesloratadine % | ND | ND | 0.03 |
| Total degradation products % | 0.03 | 0.03 | 0.06 |

## Claims

1. A solid oral stable pharmaceutical composition of Desloratadine, **characterised in that** it comprises a tablet core containing:
i) desloratadine as active substance;
ii) lactose in an amount from 12 to 50% by weight of the total weight of the tablet core;
iii) and pharmaceutically acceptable excipients;
with said core being coated with a functional polymer that acts as filmogenic agent, **characterized in that** said functional polymer is based on polyvinyl alcohol, wherein between 4% w/w and 8% w/w of the total amount of the pharmaceutical composition is the functional polymer, and wherein the oral pharmaceutical composition does not comprise either any amino acid or magnesium oxide.

2. The solid oral pharmaceutical composition of Desloratadine according to claim 1, wherein the pharmaceutically acceptable excipients are:
i) lubricants;
ii) disintegrants;
iii) and additional diluents selected from the group of microcrystalline cellulose, maize starch, mannitol and/or isomaltose.

3. The solid oral pharmaceutical composition according to claim 2, wherein the lubricant is selected form: hydrogenated cotton seed oil, sodium stearyl fumarate, magnesium stearate, calcium stearate, glyceryl palmitostearate, talc, stearic acid, glyceryl behenate, fatty acid esters, sodium lauryl sulphate, and mixtures thereof.

4. The solid oral pharmaceutical composition according to claim 2, wherein the disintegrant is selected from: low-substitution hydroxypropyl cellulose, hydroxyethyl cellulose, crospovidone, croscarmellose, pregelatinized starch, sodium starch carboxymethyl, croscarmellose sodium, soy polysaccharides, and derivatives of casein or mixtures thereof.

5. The solid oral pharmaceutical composition according to claim 2, wherein the pH modifier is selected from phosphates and citrates.

6. The solid oral pharmaceutical composition according to claims 1 to 5, wherein disintegrants are present in an amount from 5 % to 30%.

7. The solid oral pharmaceutical composition according to anyone of claims 1 to 5, **characterised in that** it also comprises:
a) 30 % to 65 % w/w of diluents other than lactose
b) 0.5-5% w/w of lubricants
c) 5-30% w/w of disintegrants
d) 0-3% w/w pH modifiers.

8. The solid oral pharmaceutical composition according to anyone of the preceding claims, **characterised in that** the ratio of the pH modifier to the disintegrant is below 0.8.

9. The solid oral pharmaceutical composition according to any of the preceding claims, wherein the average particle size, D(50), of Desloratadine is below 20 µm.

10. The solid oral pharmaceutical composition according to any of the preceding claims, wherein lactose is monohydrate lactose.

11. A process for the preparation of a solid oral pharmaceutical composition according to any of the preceding claims by direct compression comprising the steps of:
a) sieving of the components contained in the core tablet;
b) pre-mixing the active ingredient with part of the excipients, with the proviso that lactose is not included in this step;
c) mixing the blend obtained in step b) with the remaining excipients with the exception of the glidants and lubricants;
d) mixing the glidants with the blend obtained in step c);
e) mixing the lubricant with the blend obtained in step d);
f) compressing the blend obtained in step e) to form a core tablet;
g) film coating the tablet core by spaying over an aqueous or solvent dispersion of a film coating preparation.

12. The process of claim 11 wherein the same amount by weight of excipients than the active ingredient is mixed in step b).

13. The process of claim 11 wherein two lubricants are present in the tablet core **characterised in that** the additional lubricant is added in step b).

## Patentansprüche

1. Feste, orale, stabile pharmazeutische Desloratidin-Zusammensetzung, **dadurch gekennzeichnet, dass** sie einen Tablettenkern umfasst, enthaltend:
i) Desloratidin als Wirkstoff;
ii) Laktose in einer Menge von 12 bis 50 Gewichts-% von dem Gesamtgewicht des Tablettenkerns;
iii) und pharmazeutisch akzeptable Hilfsstoffe;
wobei der Kern mit einem funktionellen Polymer beschichtet ist, das als filmbildendes Mittel wirkt, **dadurch gekennzeichnet, dass** das funktionelle Polymer auf Polyvinylalkohol basiert, wobei zwischen 4 % (Gew./Gew.) und 8 % (Gew./Gew.) von der Gesamtmenge der pharmazeutischen Zusammensetzung das funktionelle Polymer ist, und wobei die orale pharmazeutische Zusammensetzung weder eine Aminosäure noch Magnesiumoxid umfasst.

2. Feste, orale pharmazeutische Desloratidin-Zusammensetzung nach Anspruch 1, wobei die pharmazeutisch akzeptablen Hilfsstoffe sind:
i) Schmiermittel;
ii) Sprengmittel;
iii) und weitere Verdünnungsmittel, ausgewählt aus der Gruppe aus mikrokristalliner Cellulose, Maisstärke, Mannitol und/oder Isomaltose.

3. Feste, orale pharmazeutische Zusammensetzung nach Anspruch 2, wobei das Schmiermittel ausgewählt ist aus: hydriertem Baumwollsamenöl, Natriumstearylfumarat, Magnesiumstearat, Calciumstearat, Glycerylpalmitostearat, Talkum, Stearinsäure, Glycerylbehenat, Fettsäureestern, Natriumlaurylsulfat, und Mischungen davon.

4. Feste, orale pharmazeutische Zusammensetzung nach Anspruch 2, wobei das Sprengmittel ausgewählt ist aus: niedrig-substituierter Hydroxypropylcellulose, Hydroxyethylcellulose, Crospovidon, Croscarmellose, vorgelierter Stärke, Natriumcarboxymethylstärke, Croscarmellose-Natrium, Sojapolysacchariden und Caseinderivaten, oder Mischungen davon.

5. Feste, orale pharmazeutische Zusammensetzung nach Anspruch 2, wobei das pH-Modifiziermittel ausgewählt ist aus Phosphaten und Citraten.

6. Feste, orale pharmazeutische Zusammensetzung nach Ansprüchen 1 bis 5, wobei die Sprengmittel in einer Menge von 5 % bis 30 % vorhanden sind.

7. Feste, orale pharmazeutische Zusammensetzung nach einem der Ansprüche 1 bis 5, **dadurch gekennzeichnet, dass** sie des Weiteren umfasst:
a) 30 % bis 65 % (Gew./Gew.) anderer Verdünnungsmittel als Laktose
b) 0,5-5 % (Gew./Gew.) Schmiermittel
c) 5-30 % (Gew./Gew.) Sprengmittel
d) 0-3 % (Gew./Gew.) pH-Modifiziermittel.

8. Feste, orale pharmazeutische Zusammensetzung nach einem der vorangehenden Ansprüche, **dadurch gekennzeichnet, dass** das Verhältnis des pH-Modifiziermittels zu dem Sprengmittel unter 0,8 ist.

9. Feste, orale pharmazeutische Zusammensetzung nach einem der vorangehenden Ansprüche, wobei die durchschnittliche Partikelgröße, D(50), von Desloratidin unter 20 µm ist.

10. Feste, orale pharmazeutische Zusammensetzung nach einem der vorangehenden Ansprüche, wobei Laktose Monohydrat-Laktose ist.

11. Verfahren zur Herstellung einer festen, oralen pharmazeutischen Zusammensetzung nach einem der vorangehenden Ansprüche durch direktes Komprimieren, umfassend die Schritte:
a) Sieben der in dem Tablettenkern umfassten Bestandteile;
b) Vormischen des Wirkstoffs mit einem Teil der Hilfsstoffe, mit der Maßgabe, dass Laktose in diesem Schritt nicht umfasst wird;
c) Mischen der in Schritt b) erhaltenen Mischung mit den restlichen Hilfsstoffen mit Ausnahme der Gleitmittel und Schmiermittel;
d) Mischen der Gleitmittel mit der in Schritt c) erhaltenen Mischung;
e) Mischen des Schmiermittels mit der in Schritt d) erhaltenen Mischung;
f) Komprimieren der in Schritt e) erhaltenen Mischung, um einen Tablettenkern zu bilden;
g) Filmbeschichten des Tablettenkerns durch Aufsprühen einer wässrigen oder Lösungsmittel-Dispersion von einer Filmbeschichtungszubereitung.

12. Verfahren nach Anspruch 11, wobei die gleiche Gewichtsmenge von Hilfsstoffen wie von dem Wirkstoff in Schritt b) gemischt wird.

13. Verfahren nach Anspruch 11, wobei zwei Schmiermittel in dem Tablettenkern vorhanden sind, **dadurch gekennzeichnet, dass** das weitere Schmiermittel in Schritt b) zugegeben wird.

## Revendications

1. Une composition pharmaceutique solide et stable par voie orale de Desloratadine, **caractérisée en ce qu'**elle comprend un noyau de comprimé contenant :
i) de la Desloratadine en tant que substance active ;
ii) du lactose en une quantité de 12 à 50% en poids du poids total du noyau du comprimé ;
iii) et des excipients pharmaceutiquement acceptables ;
ledit noyau étant revêtu d'un polymère fonctionnel qui agit comme agent filmogène,
**caractérisé en ce que** ledit polymère fonctionnel est à base d'alcool polyvinylique, dans lequel entre 4% p/p et 8% p/p de la quantité totale de la composition pharmaceutique est le polymère fonctionnel, et dans lequel la composition pharmaceutique par voie orale ne comprend ni acide aminé ni oxyde de magnésium.

2. La composition pharmaceutique solide de Desloratadine par voie orale selon la revendication 1, dans laquelle les excipients pharmaceutiquement acceptables sont :
i) des lubrifiants ;
ii) des désintégrants ;
iii) et des diluants supplémentaires choisis dans le groupe comprenant la cellulose microcristalline, l'amidon de maïs, le mannitol et/ou l'isomaltose.

3. La composition pharmaceutique solide par voie orale selon la revendication 2, dans laquelle le lubrifiant est choisi parmi : huile de graine de coton hydrogénée, fumarate de stéaryle sodique, stéarate de magnésium, stéarate de calcium, palmitostéarate de glycéryle, talc, acide stéarique, béhénate de glycéryle, esters d'acides gras, sulfate de laurylsodium, et des mélanges de ceux-ci.

4. La composition pharmaceutique solide par voie orale selon la revendication 2, dans laquelle le désintégrant est choisi parmi : l'hydroxypropylcellulose à faible substitution, l'hydroxyéthylcellulose, la crospovidone, la croscarmellose, l'amidon prégélatinisé, l'amidon carboxyméthylique sodique, le croscarmellose sodique, les polysaccharides à base de soja, et les dérivés de caséine, ou des mélanges de ceux-ci.

5. La composition pharmaceutique solide par voie orale selon la revendication 2, dans laquelle le modificateur de pH est choisi parmi les phosphates et les citrates.

6. La composition pharmaceutique solide par voie orale selon les revendications 1 à 5, dans laquelle des désintégrants sont présents en une quantité allant de 5% à 30%.

7. La composition pharmaceutique solide par voie orale selon l'une quelconque des revendications 1 à 5, **caractérisée en ce qu'**elle comprend également :
a) de 30 à 65% p/p de diluants autres que le lactose
b) de 0,5 à 5% p/p de lubrifiants
c) de 5 à 30% p/p d'agents de désintégration
d) de 0 à 3% p/p de modificateurs de pH.

8. La composition pharmaceutique solide par voie orale selon l'une quelconque des revendications précédentes, **caractérisée en ce que** le rapport du modificateur de pH au désintégrant est inférieur à 0,8.

9. La composition pharmaceutique solide par voie orale selon l'une quelconque des revendications précédentes, dans laquelle la taille moyenne des particules, D(50), de Desloratadine est inférieure à 20 µm.

10. La composition pharmaceutique solide par voie orale selon l'une quelconque des revendications précédentes, dans laquelle le lactose est du lactose monohydraté.

11. Un procédé de préparation d'une composition pharmaceutique solide par voie orale selon l'une quelconque des revendications précédentes par compression directe comprenant les étapes consistant à :
a) tamiser des composants contenus dans le comprimé de noyau ;
b) pré-mélanger le principe actif avec une partie des excipients, à condition que le lactose ne soit pas inclus dans cette étape ;
c) mélanger le mélange obtenu à l'étape b) avec les excipients restants à l'exception des agents de glissement et des lubrifiants ;
d) mélanger les agents de glissement avec le mélange obtenu à l'étape c) ;
e) mélanger le lubrifiant avec le mélange obtenu à l'étape d) ;
f) comprimer le mélange obtenu à l'étape e) pour former un comprimé de noyau ;
g) pelliculer le noyau du comprimé par projection sur une dispersion aqueuse ou de solvant d'une préparation de pelliculage.

12. Le procédé selon la revendication 11, dans lequel la même quantité en poids d'excipients que l'ingrédient actif est mélangé à l'étape b).

13. Le procédé selon la revendication 11, dans lequel deux lubrifiants sont présents dans le noyau de comprimé, **caractérisé en ce que** le lubrifiant supplémentaire est ajouté à l'étape b).
